(19) **Europäisches Patentamt / European Patent Office / Office européen des brevets**

(11) **EP 2 492 008 A1**

(12) **EUROPEAN PATENT APPLICATION**
published in accordance with Art. 153(4) EPC

(43) Date of publication:
**29.08.2012 Bulletin 2012/35**

(21) Application number: **10824825.3**

(22) Date of filing: **12.10.2010**

(51) Int Cl.:
*B01J 23/80* (2006.01)   *C07C 1/04* (2006.01)
*C07C 1/12* (2006.01)   *C07C 31/04* (2006.01)
*C07B 61/00* (2006.01)

(86) International application number:
**PCT/JP2010/067853**

(87) International publication number:
**WO 2011/048976 (28.04.2011 Gazette 2011/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.10.2009 JP 2009244541**

(71) Applicant: **Mitsubishi Gas Chemical Company,
Inc.
Tokyo 100-8324 (JP)**

(72) Inventors:
• **YAMADA, Hajime**
  **Niigata-shi**
  **Niigata 950-3112 (JP)**
• **WATANABE, Toshiyasu**
  **Niigata-shi**
  **Niigata 950-3112 (JP)**

(74) Representative: **Vossius & Partner
Siebertstrasse 4
81675 München (DE)**

(54) **METHANOL SYNTHESIS CATALYST**

(57)    A catalyst for methanol synthesis which is improved in an activity, a lifetime and a strength is provided. It is a catalyst for methanol synthesis comprising essential components of copper, zinc and alumina, wherein an atomic ratio of copper/zinc falls in a range of 1 to 3; an alumina content is 3 to 20 % by weight; an alumina source is alumina hydrate having a pseudo boehmite structure; and it is molded so that a density is 2.0 to 3.0 g/ml.

EP 2 492 008 A1

## Description

Technical Field

The present invention relates to a copper-zinc-aluminum base catalyst used principally for methanol synthesis reaction carried out by reacting hydrogen with carbon monoxide and/or carbon dioxide.

Related Art

In synthesizing methanol by reacting hydrogen with carbon monoxide and/or carbon dioxide, a copper base catalyst is used as a conventional catalyst, and various catalyst production methods are proposed in order to improve an activity and a strength of catalysts or rationalize production steps thereof.
A methanol synthetic process is a very important basic process in a chemical industry, and a high efficiency thereof is constantly required from the viewpoints of energy saving and economical efficiency thereof. One of the most important techniques in a methanol synthetic process is to provide a catalyst of a high performance, and three component catalysts such as a $Cu/ZnO/Al_2O_3$ base catalyst (an existing industri23.11al catalyst; refer to, for example, a non-patent document 1) and a $Cu/ZnO/SiO_2$ base catalyst (a patent document 1) are known as conventional catalysts.

Methanol is expected to be demanded more and more as intermediate raw materials for formalin, MTBE (methyl tertiary butyl ether) and MMA and in addition thereto, raw materials for gasoline, petrochemical intermediate products, hydrogen, carbon monoxide, city gas and the like, and large-sized methanol synthesis plants are anticipated to be constructed all over the world.
In recent trend, raw material gas for methanol synthesis is being changed to a carbon-rich raw material gas composition according to a two-stage reforming method and addition of carbon dioxide in terms of a economical efficiency and global warming, and performances of a high activity and a high lifetime which meet a wide variety of raw material gas compositions are requested to the catalysts.

In methanol synthesis from raw material gas having a high content of carbon dioxide, a catalyst having a higher activity than that of the catalyst employed in the foregoing methanol synthesis from synthetic gas is required due to thermodynamic equilibrium of reaction and a reaction inhibitory effect of water produced together with methanol (a non-patent document 2). Further, in methanol synthesis from raw material gas having a high content of carbon dioxide, damage in a catalyst activity which is estimated to be attributable to water produced together with methanol is very large as compared with methanol synthesis from synthetic gas. Accordingly, a catalyst having a much higher durability than that of the catalyst employed in the methanol synthesis from synthetic gas is required, and a durability is not necessarily satisfactory in the three component catalyst employed in the methanol synthesis described above.

From the above viewpoints, catalysts of a copper/zinc oxide/aluminum oxide/zirconium oxide base, a copper/ zinc oxide/aluminum oxide/zirconium oxide/gallium oxide base and the like are proposed (patent documents 2 to 3 and the non-patent documents 2). However, since expensive zirconium and gallium are used in the above catalysts, it results in an increase in the costs, and the existing situation is that they are scarcely used on an actually commercial basis.

Also, a high performance of a handling strength suited to a large-sized synthetic reaction vessel is required to the catalyst. Further, the structure of the reaction vessel is changed from a heat insulation type to a complicated type such as a multipipe, heat exchange type, and since powdering of the catalysts brings about troubles such as clogging, an increase in a differential pressure and the like, the high strength catalysts which can sufficiently endure use thereof are required as well.

Related Art Documents

Patent Documents

Patent document 1: Japanese Patent Application Laid-Open No. 39287/1988
Patent document 2: Japanese Patent Application Laid-Open No. 39755/1995
Patent document 3: Japanese Patent Application Laid-Open No. 312138/1994

Non-patent Documents

Non-patent document 1: Catalyst Course, vol. 7, edited by Catalysis Society of Japan, published by Kodansha Ltd.,

published on July 20, 1989, p. 21 to 39
Non-patent document 2: Applied Catalysis A: General, 38 (1996), p. 311 to 318

Outline of the Invention

Problems to be Solved by the Invention

An object of the present invention is to solve the problems described above in conventional techniques and provide a catalyst for methanol synthesis which is improved in an activity, a lifetime and a strength.

Means for Solving the Problems

Intense investigations carried out by the present inventors for the purpose of solving the problems described above have resulted in finding an optimum composition of a catalyst for methanol synthesis which can be improved in an activity, a lifetime and a strength and precipitate preparing conditions, molding conditions thereof and the like, and thus they have reached the present invention.
That is, the present invention relates to a catalyst for methanol synthesis comprising essential components of copper, zinc and alumina, wherein an atomic ratio of copper/zinc falls in a range of 1 to 3; an alumina content is 3 to 20 % by weight; an alumina source is alumina hydrate having a pseudo boehmite structure; and it is molded so that a density is 2.0 to 3.0 g/ml and a production process for methanol in which hydrogen is reacted with carbon monoxide and/or carbon dioxide in the presence of the above catalyst.

Effects of the Invention

An inexpensive catalyst for methanol synthesis which is improved in an activity, a lifetime and a strength is provided by the present invention.

Mode for Carrying Out the Invention

The present invention shall be explained below in detail.
The present inventors have intensely researched a catalyst for methanol synthesis having the problems described above, and as a result thereof, they have found that a catalyst which contains at least copper, zinc and alumina, in which alumina or alumina hydrate having a pseudo boehmite structure is used as an alumina source and which is prepared by forming copper and zinc precipitates, mixing them with an alumina source of alumina hydrate having a pseudo boehmite structure, then preferably baking the mixture and molding it on constant conditions is improved in an activity, a lifetime and a strength, and thus they have reached the present invention. That is, the present invention relates to a catalyst for methanol synthesis comprising an essential component of copper-zinc-alumina which is prepared and molded by an optimum method and a methanol production process using the above catalyst.

Catalyst for methanol synthesis:

In preparing the catalyst for methanol synthesis of the present invention, copper and zinc which are the essential components of the catalyst can be precipitated and prepared by mixing a metal salt aqueous solution containing copper and zinc with an alkali aqueous solution. It is reported that in the above case, simultaneous addition of the alkali aqueous solution and the metal salt aqueous solution is advantageous in terms of the activity.
In the present invention, the addition method may be the above method, and the alkali aqueous solution may be added to the metal salt aqueous solution or the metal salt aqueous solution may be added to the alkali aqueous solution.
Water-soluble salts such as copper nitrate, copper sulfate, copper acetate and the like can be used as the copper source in producing the catalyst for methanol synthesis of the present invention. Further, copper chloride and copper sulfate which are recovered from very inexpensive etching waste liquids can be used as well, but in the above case, a step for removing chlorine and sulfur which are catalyst poisons is necessary. Water-soluble salts such as zinc nitrate, zinc sulfate, zinc acetate and the like can be used as the zinc source. Inexpensive and water-insoluble zinc compounds such as zinc oxide, zinc hydroxide and the like may be used as well, and solutions prepared by dissolving the above compounds in acid such as sulfuric acid, nitric acid and the like may be used as well. Further, the above compounds are dispersed well in water and the like to prepare a slurry, and it may be used after carbonated by blowing carbon dioxide. A slurry of zinc oxide may be carbonated, or zinc oxide may be carbonated after mixed with a precipitation component of copper.
The contents of copper and zinc which are the active components of the catalyst of the present invention are preferably 13 to 88 % by weight and 67 to 9 % by weight, more preferably 18 to 77 % by weight and 62 to 20 % % by weight and further preferably 39 to 73 % by weight and 41 to 24 % % by weight in terms of oxides respectively based on a whole part of the catalyst from the viewpoint of an activity and a lifetime of the catalyst. Further, a composition of copper and zinc contained in the catalyst falls in a range of 0.2 to 10 : 1 (0.2 to 10 in terms of a copper/zinc ratio), preferably 0.3 to 4 : 1 (0.3 to 4 in terms of a copper/zinc ratio) and more preferably 1 : 1 to 3 (1 to 3 in terms of a copper/zinc ratio) in terms of an atomic ratio of copper : zinc. When the above ratio is larger than necessary, the initial activity is enhanced, but sintering of copper takes place as well, and the activity and the strength are reduced to a large extent. On the other hand, when it is small, the initial activity is reduced.

An alkali hydroxide aqueous solution, aqueous ammonia and the like can be used as the alkali solution for precipitating metal from the metal salt aqueous solution. In order to effectively crystallize it into basic carbonate, an alkaline aqueous solution containing carbonates such as sodium carbonate, sodium bicarbonate, ammonium carbonate, ammonium bicarbonate and the like is suitably used.

A use amount of alkali which is the precipitant is 1 to 2 times, preferably 1.1 to 1.8 times and more preferably 1.1 to 1.5 times as large as a mole equivalent based on the metal salt.

In precipitating and forming the catalyst components, time required for addition of the alkali aqueous solution or the metal salt aqueous solution each described above is 20 seconds to 3 hours, preferably 1 minute to 2 hours and more preferably 2 minutes to 60 minutes from the viewpoint of the catalyst performances (the activity, the lifetime and the strength).

Temperature for precipitating and forming is preferably 20 to 90°C, more preferably 30 to 80°C and more preferably 30 to 60°C from the viewpoint of the catalyst performances (the activity, the lifetime and the strength). In this case, a concentration of the water-soluble metal salt contained in the metal salt aqueous solution and the precipitant contained in the alkali aqueous solution falls in a range of preferably 0.2 to 3 mole/L, more preferably 0.5 to 2 mole/L.

In a copper-zinc base catalyst for methanol synthesis, ripening is preferably carried out in order to crystallize them after precipitating and forming copper and zinc, and in this case, discoloration phenomenon in which a hue of the precipitate is changed from blue to green is observed.

A blowing amount of carbon dioxide in carrying out the carbonation described above is preferably 20 to 500 L/h • kg-ZnO, more preferably 40 to 400 L/h • kg-ZnO and further preferably 100 to 300 L/h • kg-ZnO in order to effectively crystallize it into basic carbonate.

It is important to use alumina hydrate having a pseudo boehmite structure as the alumina source in producing the catalyst for methanol synthesis of the present invention. Alumina hydrate is obtained by adsorbing crystal water on aluminum oxide, and the above alumina hydrate is classified into a crystalline alumina gel and a gelatinous alumina gel. The pseudo boehmite structure is classified into a gelatinous alumina gel. The pseudo boehmite structure can be confirmed by an X ray diffraction method.

Commercial sources may be used for the alumina source which is alumina hydrate having a pseudo boehmite structure, and a production method thereof shall not specifically restricted. It is produced by a neutralization decomposition method, a hydrolysis method of aluminum alkoxide and the like.

In, for example, the neutralization decomposition method, optional aluminum salts such as aluminum sulfate, aluminum chloride, aluminum nitrate and the like can be used. Also, optional compounds such as sodium aluminate, potassium aluminate and the like can be used as aluminate. The neutralization reaction includes a method in which an alkaline aqueous solution of potassium hydroxide, aqueous ammonia and the like is added to an aluminum salt aqueous solution, a method in which an acid aqueous solution of sulfuric acid, hydrochloric acid, nitric acid and the like is added to an aluminate aqueous solution and a method in which an aluminum salt aqueous solution is mixed with an aluminate aqueous solution. In the above case, a crystallization inhibitor such as carboxylic acids and the like may be added to control precipitation.

In the hydrolysis method of aluminum alkoxide, water is added to aluminum alkoxide such as aluminum butoxide, aluminum isopropoxide and the like at high temperature such as a boiling point of a solvent to hydrolyze it, and pseudo boehmite is produced.

In the present invention, pseudo boehmite is produced preferably from an aluminum salt aqueous solution and an aluminate aqueous solution by neutralization reaction in terms of a production cost. To be specific, combination of an aluminum sulfate aqueous solution and a sodium aluminate aqueous solution is preferred.

A factor exerting an effect on the physical properties of the alumina hydrate to a large extent includes conditions in forming the precipitates and conditions in ripening the precipitates. The pH, the temperature, the aluminum concentration, the salt concentration and the time are important parameters for the above conditions. To be specific, a pH of 4 to 11 and a temperature of 30 to 60°C in the neutralization are considered to be favorable.

The physical properties of the alumina hydrate having a pseudo boehmite structure are a BET specific surface area of preferably 100 to 600 $m^2$/g, more preferably 200 to 500 $m^2$/g, a whole pore volume of preferably 0.1 to 0.8 ml/g, more preferably 0.2 to 0.6 ml/g and an average pore diameter of preferably 1 to 7 nm, more preferably 3 to 6 nm after baking at 500°C from the viewpoint of the catalyst performances (the activity, the lifetime and the strength).

The alumina hydrate having a pseudo boehmite structure which is the alumina source may be mixed with the copper and zinc components either in a step of ripening the copper and zinc precipitate slurries or after finishing the ripening step. In the above case, even the dried product can be added by turning it into a slurry in water. A method in which precipitates of three components of the copper, zinc and alumina sources are formed by a coprecipitation method and mixed is available as well, but a method preferably separating formation of precipitates of copper and zinc from addition of the alumina source which is the alumina hydrate having a pseudo boehmite structure and mixing is preferable from the viewpoint of the catalyst performances (the activity, the lifetime and the strength).

A mixing temperature of the alumina hydrate having a pseudo boehmite structure with the copper and zinc components can be selected in a range of 90°C or lower, and it is preferably 0 to 85°C, more preferably 30 to 80°C from the viewpoint of the catalyst performances (the activity, the lifetime and the strength).

A content of alumina in the catalyst for methanol synthesis of the present invention is preferably 1 to 30 % by weight, more preferably 2 to 25 % by weight and further preferably 3 to 20 % by weight in terms of alumina in the state of oxides of copper, zinc and alumina as the form of the catalyst after baked. If it is too small, the lifetime extension effect exerted by a dilution effect of alumina is scarcely expected, and if it is too large, a content of copper which is the active component is decreased, so that the activity is reduced.

Boron and silicon compounds and magnesium compounds in addition to copper, zinc and alumina can further be added to the catalyst for methanol synthesis of the present invention in order to improve an activity of the catalyst and enhance a strength thereof. The magnesium compound and the silicon compound can be added as well in a kneading step described later, and an addition timing thereof can suitably be selected.

Boric acid, borax and the like can be used as the boron source. Further, silicon oxide and a precursor of silicon oxide can be used as the silicon source. In particular, double decomposition products of sodium silicate and diatomaceous earth are advantageous.

Water-soluble salts such as magnesium nitrate, magnesium sulfate, magnesium acetate and the like can be used, to be specific, as the magnesium source. Further, magnesium oxide, basic magnesium carbonate can be used as well, and they may be mixed with the metal salt aqueous solution of copper and zinc to form precipitates by the alkali aqueous solution, or they may be added together with the silicon source. An addition amount thereof is usually 0.1 to 10 % by weight, preferably 0.2 to 5 % by weight and more preferably 0.3 to 3 % by weight in terms of oxide based on an oxide state ($CuO/ZnO/Al_2O_3$).

Further, precursors of oxides of Zr, La, Mn, Cr and the like, for example, carbonates and the like and oxygen acid salts of phosphorus can be added, if necessary, to the catalyst for methanol synthesis of the present invention.

A mixed slurry obtained by the above operation is usually filtrated, and then acidic ions and alkali ions are removed by washing with water. The silicon source and the magnesium source can be added, if necessary, to the composition thus produced. When the composition is a cake, a conventional mixer, for example, a kneading equipment, a reciprocatory stirring equipment and the like can be used.

The cake or the slurry thus obtained is dried at a temperature of, for example, 50 to 150°C and then baked in a temperature range of preferably 180 to 500°C, more preferably 200 to 450°C under an aerial atmosphere, and then it is crushed by a publicly known method and passes through a molding step, whereby the catalyst is prepared.

A lubricant such as graphite and the like is added, if necessary, to the catalyst obtained after baked, and it is molded by means of a perforated plate and a pelletizing equipment and then can be used. Also, crushing treatment may be carried out, if necessary, before molding.

In general, an amount of an active component per a unit volume can be raised by elevating a density of a molded article, and therefore a catalyst having a high activity is obtained. However, if the density is elevated too much, pores of an active part are broken, and the active component is reduced in a specific surface area, or the products are prevented from being diffused, so that the activity and the lifetime are rather reduced in a certain case. Accordingly, in the present invention, the catalyst has to be molded so that an optimum density is obtained.

A strength of the catalyst molded article is important. If it is too weak, the catalyst is crushed to reduce to powder in a charging work and a catalyst exchange work in an actual equipment, and if it is too strong, the catalyst is fragile and liable to be cracked. If the above phenomenon takes place, an increase in a differential pressure in operating the apparatus is brought about, and clogging of the apparatus is likely to be caused in the worst case.

Powdering of the catalyst is brought about principally in a charging work of the catalyst in an actual equipment and a catalyst exchange work. In the above case, if the powdering rate is large, the workers are likely to aspire the powder and concerned about in terms of health. Also in terms of operating the actual equipment, if the powder is large, a differential pressure of the reactor is increased, and the cases of clogging of the apparatus, an increase in the differential pressure caused by carrying the powder to a downstream, clogging of the pipelines and mixing in the products are likely to be brought about. Accordingly, it is important as well in the present invention to control the powdering rate, and molding has to be carried out while keeping a balance between a strength and a powdering rate of the catalyst.

In the present invention, the optimum values of the activity, the lifetime and the strength of the catalyst have been found by controlling the density, the specific surface area, the pore volume and the pore diameter of the molded article. From the viewpoints described above, a density of the catalyst molded article is preferably 1.0 to 4.0 g/ml, more preferably 1.5 to 3.5 g/ml and further preferably 2.0 to 3.0 g/ml.

Also, a specific surface area of the catalyst molded article is preferably 10 to 300 $m^2$/g, more preferably 50 to 200 $m^2$/g, further preferably 50 to 150 $m^2$/g and particularly preferably 80 to 120 $m^2$/g. A whole pore volume thereof is preferably 0.1 to 0.5 ml/g, more preferably 0.2 to 0.4 ml/g and further preferably 0.2 to 0.35 ml/g. An average pore diameter thereof is preferably 5 to 20 nm, more preferably 5 to 15 nm and further preferably 8 to 12 nm.

In the present invention, the precipitate preparing method, drying and baking conditions and the tableting and molding conditions are suitably controlled to mold the catalyst so that the density described above is obtained, whereby the catalyst molded article which is improved in an activity, a lifetime and a strength and which is easy to handle in an actual industrial plant can be provided.

The molded article is used in an actual industrial plant, and it is considered to be very important to evaluate the catalyst having the above form.

In the present invention, it shall not be restricted to add an activity improving agent and a durability improving agent for the catalyst and a strength improving agent aiming at further improving a strength thereof. It shall not be restricted to add, for example, compounds of Si, Ce, La, Nd, Zr, B, Mg, Ga, Cr, Sc, Y, Ca, Sr, Ba, Ra, Be, Pd, Mn, In, Nb, V, P and the like, acetic acid, oxypolycarboxylic acids, diatomaceous earth and the like. To be specific, silicon oxide and silicon oxide precursors can be used as the Si source, and sodium silicate double decomposition products and diatomaceous earth are particularly advantageous. Capable of being used as the Ce, La and Nd sources are oxides and oxide precursors thereof, for example, salts of nitric acid, sulfuric acid, acetic acid, oxalic acid, carbonic acid and the like. Zirconium oxide or oxide precursors thereof, for example, zirconium or zirconyl salts of nitric acid, sulfuric acid and the like can be used as the Zr source. Boron oxide, boric acid and the like can be used as the B source. The components described above may be used alone or in combination of a plurality thereof.

The catalyst for methanol synthesis of the present invention can be used for the following methanol synthetic reaction, and in addition thereto, it can also be used as catalysts for carbon monoxide conversion reaction, hydrogenation reaction, cracking of methanol and steam reforming reaction.

Production process for catalyst for methanol synthesis:

The catalyst for methanol synthesis of the present invention is obtained preferably by a production process for a catalyst for methanol synthesis in which an atomic ratio of copper/zinc falls in a range of 1 to 3 and an alumina content is 3 to 20 % by mass, comprising (1) a step for mixing at least an aqueous solution containing copper, an aqueous solution containing zinc and an alkali aqueous solution to form a precipitate containing copper and zinc, (2) a step for mixing the precipitate obtained with an alumina hydrate having a pseudo boehmite structure and (3) a step for molding the mixture obtained so that a density thereof is 2.0 to 3.0 g/ml.

The respective details of the steps (1) to (3) have been described above.

Production process for methanol:

The catalyst for methanol synthesis of the present invention, for example, after subjected to activation treatment by reduction with hydrogen, carbon dioxide and the like, is used for reaction of synthesizing methanol from a mixed gas of hydrogen with carbon dioxide and/or carbon monoxide. That is, in the production process for methanol according to the present invention, hydrogen is reacted with carbon dioxide and/or carbon monoxide in the presence of the catalyst for methanol synthesis of the present invention. The above methanol synthetic reaction can be carried out under an applied pressure of 20 to 300 atmospheric pressure, preferably 30 to 150 atmospheric pressure at a temperature of 150 to 350°C, preferably 200 to 300°C and a gas space velocity of 2,000 to 50,000 /h.

Examples

The present invention shall specifically be explained below in examples and comparative examples, but the present invention shall not be restricted in a scope thereof by these examples.

Example 1

Sodium aluminate 302 g (containing 18.9 wt % of alumina) was dissolved in ion-exchanged water 2000 g and kept at a temperature of 40°C, and this solution was designated as a solution A. An aluminum sulfate aqueous solution 422 g (containing 8 wt % of alumina) was dissolved in ion-exchanged water 500 g and kept at a temperature of 40°C, and this solution was designated as a solution B. The solution A and the solution B were mixed and stirred to carry out precipitation reaction. Then, the mixture was filtrated and subsequently washed several times with 3000 g of ion-exchanged water of 40°C to obtain alumina hydrate. This hydrate was dried at 120°C for a day and measured by an X ray diffraction method to find that it had a pseudo boehmite structure. Also, a BET specific surface area, a whole pore volume and an average pore diameter of the alumina hydrate which was obtained above and baked at 500°C were 414 $m^2$/g, 0.45ml/g and 4.6 nm respectively .

Copper sulfate pentahydrate 315 g and boric acid 20 g were dissolved in ion-exchanged water 1000 g and kept at a temperature of 40°C, and this solution was designated as a solution C. Sodium carbonate 162 g was dissolved in ion-exchanged water 1000 g and kept at a temperature of 40°C, and this solution was designated as a solution D. Zinc oxide 52 g was dispersed and suspended in ion-exchanged water 300 g and kept at a temperature of 40°C, and this solution was designated as a solution E.

The foregoing alumina hydrate having a pseudo boehmite structure was sampled so that alumina was 7.9 g, and it was dispersed well in ion-exchanged water 500 g and turned into a slurry. The slurry was kept at a temperature of 40°C and designated as a solution F.

The solution C was added to the solution D under stirring, and then the solution E was added thereto. Carbon dioxide was immediately blown into the solution at a rate of 200 L/h • kg-ZnO. The solution was kept at a temperature of 40°C and further heated up to 80°C, and it was kept for 30 minutes and then cooled down to 60°C. The solution F was added to this slurry and stirred for 20 minutes. Then, the slurry was filtrated, and subsequently the cake was washed several times with 3000 g of a 0.04 % sodium hydroxide aqueous solution. Further, it was washed with 3000 g of ion-exchanged water to obtain a composition cake. Diatomaceous earth (Celite Corporation, filter cell) 1.5 g, ion-exchanged water 200 g and acetic acid 4 g were added to the above cake, and they were mixed by means of a reciprocatory stirring device. Then, it was dried at 80°C for 12 hours under an aerial atmosphere. This dried product was baked at 380°C under air flow and sieved to 16 mesh or less, and graphite 3 % was added thereto to prepare a raw material powder for molding. This raw material powder was tableted to a form of 6 mm $\phi \times$ 5 mm H by molding so that a density was 2.2 g/ml. The respective properties of the molded article thus obtained were evaluated. The results thereof are shown in Table 1.

Comparative Example 1

An aluminum sulfate aqueous solution (containing 8 % of alumina) 422 g was dissolved in ion-exchanged water 500 g and kept at a temperature of 60°C, and this solution was designated as a solution G. Sodium hydroxide 92 g was dissolved in ion-exchanged water 500 g and kept at a temperature of 60°C, and this solution was designated as a solution H. The solution G and the solution H were mixed and stirred to carry out precipitation reaction. Then, the precipitate was filtrated and subsequently washed several times with 3000 g of ion-exchanged water of 40°C to obtain alumina hydrate. This hydrate was dried at 120°C for a day and measured by an X ray diffraction method to find that it had a crystalline aluminum hydroxide structure.

A catalyst was prepared in the same manner as in Example 1, except that the alumina hydrate having a crystalline aluminum hydroxide structure which was prepared from the aluminum sulfate aqueous solution and sodium hydroxide was used as the alumina source in place of the alumina hydrate having a pseudo boehmite structure. The respective properties of the molded article thus obtained were evaluated. The results thereof are shown in Table 1.

Comparative Example 2

A catalyst was prepared in the same manner as in Example 1, except that the alumina hydrate having a pseudo boehmite structure was not used as the alumina source and that 7.9 g of a product obtained by crushing commercial γ-alumina to 5 $\mu$m or less was added to the composition cake together with diatomaceous earth. The respective properties of the molded article thus obtained were evaluated. The results thereof are shown in Table 1.

Comparative Example 3

A catalyst was prepared by producing a raw material powder for molding in the same manner as in Example 1 and tableting it by molding so that a bulk density was 1.7 g/ml. The respective properties of the molded article thus obtained were evaluated. The results thereof are shown in Table 1.

Comparative Example 4

A catalyst was prepared by producing a raw material powder for molding in the same manner as in Example 1 and tableting it by molding so that a bulk density was 3.1 g/ml. The respective properties of the molded article thus obtained were evaluated. The results thereof are shown in Table 1.

Example 2

Zinc oxide 40 g, sulfuric acid 54 g and ion-exchanged water 240 g were added and stirred well to prepare a solution A.

Copper sulfate pentahydrate 346 g and boric acid 15 g were dissolved in ion-exchanged water 1000 g and kept at a temperature of 40°C, and this solution was designated as a solution B. Sodium carbonate 189 g was dissolved in ion-exchanged water 1000 g and kept at a temperature of 40°C, and this solution was designated as a solution C. The alumina hydrate having a pseudo boehmite structure obtained in Example 1 was sampled so that an alumina content was 7.9 g, and ion-exchanged water 500 g was added thereto. The solution was kept at a temperature of 40°C and stirred, and this solution was designated as a solution D.

The solution A and the solution B were put in a 5 L flask and kept at a temperature of 40°C while stirring, and the solution C was added thereto in 10 minutes to form a precipitate. Next, the solution D was added thereto, and then the solution was kept at a temperature of 40°C while blowing carbon dioxide at a flow rate of 220 L/h • kg-ZnO. Further, it was heated up to 80°C and kept for 30 minutes.

Then, the slurry was filtrated, and subsequently the cake was washed several times with 3000 g of a 0.02 % sodium carbonate aqueous solution. Further, it was washed with 3000 g of ion-exchanged water to obtain a composition cake. Diatomaceous earth (Celite Corporation, filter cell) 1.5 g, ion-exchanged water 200 g and acetic acid 4 g were added to the above cake, and they were mixed by means of a reciprocatory stirring device. Then, it was dried at 80°C for 12 hours under an aerial atmosphere. This dried product was baked at 380°C under air flow and sieved to 16 mesh or less, and graphite 3 % was added thereto to prepare a raw material powder for molding. This raw material powder for molding was tableted to a form of 6 mm $\phi \times$ 5 mm H by molding so that a density was 2.2 g/ml. The respective properties of the molded article thus obtained were evaluated. The results thereof are shown in Table 1.

Comparative Example 5

A catalyst was prepared in the same manner as in Example 2, except that the alumina hydrate having a pseudo boehmite structure was not used as the alumina source and that 7.9 g of a product obtained by crushing commercial $\alpha$-alumina to 5 $\mu$m or less was added to the composition cake together with diatomaceous earth. The respective properties of the molded article thus obtained were evaluated. The results thereof are shown in Table 1.

Example 3

Zinc oxide 40 g was dissolved in sulfuric acid 54 g, and then ion-exchanged water 240 g was added thereto and stirred well to prepare a solution A. Copper sulfate pentahydrate 346 g and boric acid 15 g were dissolved in ion-exchanged water 830 g to prepare a solution B. Sodium carbonate 189 g was dissolved in ion-exchanged water 1000 g and kept at a temperature of 40°C to prepare a solution C.

The alumina hydrate having a pseudo boehmite structure obtained in Example 1 was sampled so that an alumina content was 17 g, and ion-exchanged water 500 g was added thereto, and the solution was kept at a temperature of 40°C and stirred to prepare a solution D.

The solution A and the solution B were put in a 5 L flask and kept at a temperature of 40°C while stirring, and the solution C was added thereto in 10 minutes to form a precipitate. Next, the solution D was added thereto, and then the solution was kept at a temperature of 40°C while blowing carbon dioxide at a flow rate of 250 L/h • kg-ZnO. Further, it was heated up to 80°C and kept for 30 minutes.

Next, the slurry was filtrated, and subsequently the cake was washed several times with 3000 g of a 0.02 % alkali carbonate aqueous solution. Further, it was washed with ion-exchanged water. After washing, it was dehydrated to obtain a catalyst precursor cake. Diatomaceous earth (Celite Corporation, filter cell) 1.5 g, ion-exchanged water 200 g and acetic acid 4 g were added thereto, and they were mixed by stirring. Then, it was dried at 80°C for 12 hours under an aerial atmosphere and further baked at 380°C for 2 hours under air flow. After baked, it was sieved to 16 mesh or less by means of a Fitz mill sieving equipment, and graphite 3 % was added thereto to prepare a raw material powder for molding. This raw material powder for molding was tableted to a cylindrical form of 6 mm $\phi \times$ 5 mm H by molding so that a density was 2.2 g/ml. The respective properties of the molded article thus obtained were evaluated. The results thereof are shown in Table 1.

Comparative Example 6

A catalyst was prepared in the same manner as in Example 3, except that the alumina hydrate having a pseudo boehmite structure was not used as the alumina source and that 13 g of commercial crystalline aluminum hydroxide was added to the composition cake together with diatomaceous earth. The respective properties of the molded article thus obtained were evaluated. The results thereof are shown in Table 1.

Comparative Example 7

A catalyst was prepared by preparing a raw material powder for molding in the same manner as in Example 3 and tableting it by molding so that a bulk density was 1.7 g/ml. The respective properties of the molded article thus obtained were evaluated. The results thereof are shown in Table 1.

Comparative Example 8

A catalyst was prepared by preparing a raw material powder for molding in the same manner as in Example 3 and tableting it by molding so that a bulk density was 4.2 g/ml. The respective properties of the molded article thus obtained were evaluated. The results thereof are shown in Table 1.

Comparative Example 9

A catalyst was prepared in the same manner as in Example 3 except that the alumina hydrate having a pseudo boehmite structure was added so that an alumina content was 80 g. The respective properties of the molded article thus obtained were evaluated. The results thereof are shown in Table 1.

The respective properties of the molded articles thus obtained were evaluated by the following methods.

[Specific surface area, whole pore volume and average pore diameter of the catalysts]

A specific surface area, a whole pore volume and an average pore diameter of the respective catalysts obtained above were measured and evaluated by a nitrogen adsorption method (multipoint method).
AutoSorb manufactured by Yuasa Ionics Inc. was used for the nitrogen adsorption method.

[Strength of catalyst and Powdering rate of catalyst]

A strength of the respective catalysts described above was evaluated by two items of a crush strength and a powdering rate.

Strength

The crush strength was measured by means of a small-sized material test device (model PSP-100, manufactured by Fujii Seisakusho Co., Ltd.

Powdering rate

The foregoing catalyst 10 g after reduction was put in a cylindrical drum of 100 $\phi$ in which a metal gauze of JIS 6 mesh was provided on a circumference, and this drum was rotated at 150 rpm for 20 minutes to determine the powdering rate from an amount of the catalyst remaining in the drum according the following equation.

$$\text{powdering rate (\% by weight)} = [\text{amount (g) of the sample taken - remaining amount (g) in the drum}]/[\text{amount (g) of the sample taken}] \times 100$$

The crush strength and the powdering rate are shown in Table 1.

[Activity test of catalyst]

Reduction treatment for measuring an activity of the catalyst was carried out with $H_2/N_2$ = 15/85 % gas at 140°C. Then, in an activity test of methanol synthesis, a mixed gas containing 65 % of hydrogen, 7 % of carbon monoxide, 9 % of carbon dioxide, 18 % of methane and 1 % of nitrogen was used to carry out the test at a reaction pressure of 8.8 MPaG, a gas space velocity of 30,000/h and a reaction temperature of 240 to 250°C. Further, in order to determine a durability of the catalyst, methanol synthesis was carried out at a catalyst temperature of 360°C for 10 hours, and an activity thereof at 240 to 250°C was measured again. An activity of the catalyst was evaluated by a concentration (mol %) of methanol contained in an outlet gas.
Table 1

| | Cu/Zn atomic ratio | Alumina content | Form of alumina source | Bulk density | Activity of catalyst | | | | Strength | Powdering rate | Specific surface area | Whole pore volume | Average pore diameter |
| | | | | | Initial activity | | After 360°C × 10 hours | | | | | | |
| | | wt % | | g/ml | 240°C | 250°C | 240°C | 250°C | kg/cm$^2$ | wt% | m$^2$/g | ml/g | nm |
| Example 1 | 2.0 | 5 | pseudo boehmite | 2.2 | 6.0 | 7.5 | 4.2 | 5.2 | 205 | 2.0 | 110 | 0.28 | 10.2 |
| Comparative Example 1 | 2.0 | 5 | Crystallize aluminum hydroxide | 2.2 | 5.8 | 7.2 | 3.5 | 4.8 | 202 | 2.2 | 95 | 0.24 | 9.9 |
| Comparative Example 2 | 2.0 | 5 | γ-alumina | 2.2 | 4.0 | 5.0 | 2.0 | 3.1 | 180 | 9.9 | 118 | 0.31 | 10.8 |
| Comparative Example 3 | 2.0 | 5 | pseudo boehmite | 1.7 | 5.2 | 6.4 | 3.1 | 4.2 | 120 | 9.8 | 120 | 0.37 | 13.0 |
| Comparative Example 4 | 2.0 | 5 | pseudo boehmite | 3.1 | 5.9 | 7.5 | 3.2 | 4.5 | 260 | 12.2 | 85 | 0.17 | 7.4 |
| Example 2 | 2.8 | 5 | pseudo boehmite | 2.2 | 6.8 | 8.2 | 5.2 | 6.2 | 196 | 1.4 | 102 | 0.31 | 10.1 |
| Comparative Example 5 | 2.8 | 5 | α-alumina | 2.2 | 4.5 | 5.7 | 3.4 | 4.3 | 190 | 8.0 | 95 | 0.37 | 7.7 |
| Example 3 | 2.8 | 10 | pseudo boehmite | 2.2 | 6.8 | 8.3 | 5.4 | 6.5 | 214 | 1.5 | 108 | 0.27 | 10.9 |
| Comparative Example 6 | 2.8 | 10 | Crystalline aluminum hydroxide | 2.2 | 6.2 | 7.3 | 4.4 | 5.3 | 192 | 2.0 | 106 | 0.26 | 9.5 |
| Comparative Example 7 | 2.8 | 10 | pseudo boehmite | 1.7 | 6.2 | 7.6 | 4.8 | 5.2 | 110 | 8.5 | 128 | 0.40 | 13.3 |
| Comparative Example 8 | 2.8 | 10 | pseudo boehmite | 4.2 | 7.0 | 8.5 | 5.1 | 6.3 | 263 | 20.1 | 77 | 0.15 | 7.0 |
| Comparative Example 9 | 2.8 | 40 | pseudo boehmite | 2.2 | 5.9 | 7.0 | 3.5 | 4.2 | 210 | 4.5 | 145 | 0.39 | 13.7 |

**Claims**

1.  A catalyst for methanol synthesis comprising essential components of copper, zinc and alumina, wherein an atomic ratio of copper/zinc falls in a range of 1 to 3; an alumina content is 3 to 20 % by weight; an alumina source is alumina hydrate having a pseudo boehmite structure; and it is molded so that a density is 2.0 to 3.0 g/ml.

2.  The catalyst for methanol synthesis according to claim 1, wherein the alumina hydrate having a pseudo boehmite structure has a specific surface area of 200 to 500 m²/g and a whole pore volume of 0.2 to 0.6 ml/g after baked at 500°C.

3.  The catalyst for methanol synthesis according to claim 1 or 2, which is prepared via a step in which carbon dioxide is blown in a range of 20 to 500 L/h • kg-ZnO.

4.  A catalyst molded article for methanol synthesis comprising the catalyst for methanol synthesis according to any of claims 1 to 3.

5.  A production process for a catalyst for methanol synthesis in which an atomic ratio of copper/zinc falls in a range of 1 to 3 and an alumina content is 3 to 20 % by mass, comprising (1) a step for mixing at least an aqueous solution containing copper, an aqueous solution containing zinc and an alkali aqueous solution to form a precipitate containing copper and zinc, (2) a step for mixing the precipitate obtained with an alumina hydrate having a pseudo boehmite structure and (3) a step for molding the mixture obtained so that a density thereof is 2.0 to 3.0 g/ml.

6.  A production process for methanol in which hydrogen is reacted with carbon monoxide and/or carbon dioxide in the presence of the catalyst for methanol synthesis according to any of claims 1 to 3.

<div align="center">**INTERNATIONAL SEARCH REPORT**</div>

| | International application No. |
|---|---|
| | PCT/JP2010/067853 |

**A. CLASSIFICATION OF SUBJECT MATTER**
$B01J23/80(2006.01)i$, $C07C1/04(2006.01)i$, $C07C1/12(2006.01)i$, $C07C31/04$
$(2006.01)i$, $C07B61/00(2006.01)n$

According to International Patent Classification (IPC) or to both national classification and IPC

**B. FIELDS SEARCHED**

Minimum documentation searched (classification system followed by classification symbols)
B01J23/80, C07C1/04, C07C1/12, C07C31/04, C07B61/00

Documentation searched other than minimum documentation to the extent that such documents are included in the fields searched
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2008/126743 A1 (Idemitsu Kosan Co., Ltd.), 23 October 2008 (23.10.2008), paragraphs [0005], [0006], [0012], [0013] & US 2010/0112397 A & EP 2135673 A1 & CA 2681411 A & CN 101652176 A & KR 10-2009-0128460 A | 1-4,6 |
| Y | JP 63-211246 A (Mitsubishi Gas Chemical Co., Inc.), 02 September 1988 (02.09.1988), claims; page 3, upper left column, lines 4 to 11; page 3, upper left column, line 17 to page 3, upper right column, line 15 & US 4956392 A & GB 2202531 A & GB 8804573 A0 & DE 3806155 A & AU 1233288 A & CA 1303067 A & AU 612624 B | 1-4,6 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 December, 2010 (14.12.10) | 28 December, 2010 (28.12.10) |

| Name and mailing address of the ISA/ | Authorized officer |
|---|---|
| Japanese Patent Office | |
| Facsimile No. | Telephone No. |

**INTERNATIONAL SEARCH REPORT**

| International application No. |
| --- |
| PCT/JP2010/067853 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 6-320000 A (Mitsubishi Gas Chemical Co., Inc.), 22 November 1994 (22.11.1994), claims 1 to 3; paragraph [0024] (Family: none) | 3,4,6 |
| A | JP 2005-520689 A (Sud-Chemie, Inc.), 14 July 2005 (14.07.2005), paragraphs [0002], [0013], [0020], [0022] & US 6693057 B1 & US 6627572 B1 & US 7064097 B1 & EP 1487578 A & WO 2003/082468 A1 & WO 2004/060556 A1 | 1-6 |
| A | JP 2003-507296 A (Akzo Nobel N.V.), 25 February 2003 (25.02.2003), entire text & US 6503867 B1 & US 2006/0096891 A1 & EP 1204596 A & EP 1491500 A2 & WO 2001/012551 A2 & DE 60015352 D & DE 60015352 T & BR 13136 A & CA 2381410 A & AT 280737 T & ES 2232499 T & PT 1204596 E & CN 1368937 A & DK 1204596 T | 1-6 |

## REFERENCES CITED IN THE DESCRIPTION

**Patent documents cited in the description**

- JP 63039287 A
- JP 7039755 A

- JP 6312138 A

**Non-patent literature cited in the description**

- Catalyst Course. Kodansha Ltd, 20 July 1989, vol. 7, 21-39

- *Applied Catalysis A: General,* 1996, vol. 38, 311-318